# EUROPÄISCHE PATENTSCHRIFT

(11) **EP 3 370 687 B1**
(45) Veröffentlichungstag und Bekanntmachung des Hinweises auf die Patenterteilung: **05.02.2020**
(21) Anmeldenummer: 16782060.4
(22) Anmeldetag: 17.10.2016
(51) Int. Cl.: A61K 8/37, A61Q 19/00, A61K 8/04, A61K 8/06

(54) **KOSMETISCHER SCHAUM AUS EINER EMULSION ENTHALTEND GLYCERYLSTEARATCITRAT**
COSMETIC FOAM FROM AN EMULSION CONTAINING GLYCERYL STEARATE CITRATE
MOUSSE COSMÉTIQUE À PARTIR D'UNE ÉMULSION CONTENANT DU GLYCÉRYL STÉARATE CITRATE

(30) Priorität: 04.11.2015 DE 102015221565
(43) Veröffentlichungstag der Anmeldung: 12.09.2018
(73) Patentinhaber: Beiersdorf AG, 20253 Hamburg (DE)
(72) Erfinder: SKUBSCH, Kerstin, 25497 Prisdorf (DE); MÜLLER, Claudia, 25499 Tangstedt (DE); RUPP, Katrin, 22417 Hamburg (DE); ECKERT, Julia, 22303 Hamburg (DE); SCHULZ, Sabine, 22159 Hamburg (DE)
(86) Internationale Anmeldenummer: PCT/EP2016/074832
(87) Internationale Veröffentlichungsnummer: WO 2017/076614

(56) Entgegenhaltungen:
- WO-A1-2012/146576
- DE-A1-102009 048 976
- US-A1- 2013 243 836

## Beschreibung

Die vorliegende Erfindung betrifft einen kosmetischen Schaum aus einer Emulsion enthaltend Glycerylstearatcitrat und einem die Emulsion aufschäumenden Gas oder Gasgemisch aus Propan, n-Butan und/oder Isobutan, wobei die Emulsion frei ist von Polyethylenglycol-Derivaten (PEG-Derivaten). Die Erfindung betrifft ferner eine Aerosoldose mit Entnahmeventil enthaltend einen derartigen Schaum.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte bestehen (ebenso wie einige Hautreinigungsprodukte) in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion, z. B. Butter) handelt es sich um das umgekehrte Prinzip, wobei der Grundcharakter hier durch das Öl bestimmt wird.

Neben der "klassischen" Anwendung, Emulsionen direkt aus dem Vorratsbehältnis auf die Haut aufzutragen, gibt es auch eine geringere Anzahl von Anwendungen, bei denen die Emulsion zur Anwendung auf der Haut mit Hilfe eines Treibgases aufgeschäumt wird. Aufgeschäumte Emulsionen weisen aufgrund ihrer Schaumkonsistenz ein besonderes Hautgefühl auf, dass von den Anwendern als besser auf der Haut verteilbar wahrgenommen wird mit einem leichteren und weniger klebrigen Hautgefühl. Die Anwendung der moussigen Textur macht den Anwendern außerdem Spaß.

Nachteilig am Stande der Technik ist jedoch der Umstand, dass man für die Herstellung kosmetischer Emulsionsschäume den Zubereitungen Polyethylenglycole und/oder Polyethylenglycol-Derivate (PEG-Derivate) zusetzen muss (dies sind Verbindungen mit Alkohol- oder Säurefunktion, die mit Polyethylenglycolen ganz oder teilweise verethert oder verestert sind, um den Schaum über einen längeren Zeitraum stabil zu halten. Diese PEG-Derivate sind beim Verbraucher jedoch zunehmend unerwünscht, da ihnen von einigen Wissenschaftlern die gesundheitliche Unbedenklichkeit abgesprochen wird. Ob diese Bedenken wissenschaftlich begründet sind, kann im Rahmen der vorliegenden Erfindung dahingestellt bleiben. Tatsache ist hingegen, dass der Verbraucher zunehmend nach Kosmetika verlangt, die "PEG frei" sind.

Es war daher die Aufgabe der vorliegenden Erfindung, einen sensorisch attraktiven, über einen längeren Zeitraum stabilen kosmetischen Schaum auf der Basis einer aufgeschäumten Emulsion zu entwickeln, die "PEG frei" ist.

Überraschend gelöst wird die Aufgabe durch einen kosmetischen Schaum gemäß Anspruch 1.

Der erfindungsgemäße Schaum ist überraschend gleichmäßig feinblasig. Er bildet ein so genanntes Mousse.

Zwar kennt der Stand der Technik die EP1277455, EP1014916 und die DE 10138495, doch konnten diese Schriften nicht den Weg zur vorliegenden Erfindung weisen. Darüber hinaus kennt der Fachmann die WO2012/146576, US2013/243836 und DE102009048976, die ebenfalls nicht den Weg zur vorliegenden Erfindung weisen konnten.

Es ist erfindungsgemäß, wenn die Emulsion frei ist von Seifen und Tensiden mit einem HLB-Wert von größer 12. Dabei werden unter "Seiten" erfindungsgemäß Natrium- oder Kaliumsalze von Fettsäuren verstanden.

Dieser Sachverhalt ist umso überraschender, da Seifen und Tenside üblicherweise zur Stabilisierung von kosmetischen Schäumen erforderlich sind.

In dieser erfindungsgemäß vorteilhaften Ausführungsform sind die erfindungsgemäßen Schäume überraschend stabiler als unter Zusatz dieser oberflächenaktiven Verbindungen und verfügen über ein signifikant geringeres Hautreizungspotential.

Es ist erfindungsgemäß vorteilhaft, wenn die erfindungsgemäße Emulsion Glycerylstearateitrat in einer Menge von 0,1 bis 5 Gew.-%, bezogen auf das Gesamtgewicht der Emulsion und erfindungsgemäß bevorzugt in einer Menge von 1 bis 3 Gew.-%, bezogen auf das Gesamtgewicht der Emulsion enthält.

Erfindungsgemäß vorteilhafte Ausführungsformen der vorliegenden Erfindung sind dadurch gekennzeichnet, dass der Schaum gebildet wird aus 90-96 Gew.-% Emulsion und 4 bis 10 Gew.-% Gas bzw. Gasgemisch. Erfindungsgemäß bevorzugte Ausführungsformen sind dadurch gekennzeichnet, dass der Schaum gebildet wird aus 92-94 Gew.-% Emulsion und 6 bis 8 Gew.-% Gas bzw. Gasgemisch. Dass sich die erfindungsgemäßen Schäume bereits mit einer derartig geringen Menge an Gas(en) gleichmäßig aufschäumen lassen, war dabei für den Fachmann nicht vorhersehbar. Darüber hinaus sind derartige Mischungen aus Gas(en) und Emulsion überraschender Weise nicht brennbar, weshalb bei der Lagerung und Anwendung auf Brandschutzmaßnahmen verzichtet werden kann.

Es ist erfindungsgemäß vorteilhaft, wenn als Gas ein Gasgemisch aus Butan, Iso-Butan und/oder Propan eingesetzt wird. Das Mischungsverhältnis der Gase variiert je nach Druckstufe (die Druckstufe gibt also das Mischungsverhältnis der Gase an), siehe z.B: Drivosol 27 D48, Drivosol 27 D60, Drivosol 30 A und Drivosol 35 A von Evonik mit 5-60 % Butan, 15-25% Propan und 20-80 % Iso-Butan. Erfindungsgemäß bevorzugt sind die Druckstufen 2,7 / 3,0/ 3,5 bar. Erfindungsgmeäß besonders bevorzugt sind die Druckstufen 3,0 bar und 3,5 bar.

Druckstufe 2,7 bar: 60 % Butan, 20% Propan und 20 % Iso-Butan.
Druckstufe 3,0 bar: 5,3 % Butan, 15,3% Propan und 79,4 % Iso-Butan.
Druckstufe 3,5 bar: 5 % Butan, 23% Propan und 72 % Iso-Butan.

Für den Herstellprozess ist es von Vorteil, wenn das Füllgut eine gewisse Lagerstabilität aufweist, falls nicht sofort nach Herstellung die Aerosolabfüllung erfolgen kann. Üblicherweise haben die Füllgüter für Aerosolprodukte keine Lagerstabilität.
Die erfindungsgemäßen Formulierungen haben den Vorteil, als noch nicht abgefülltes Füllgut eine relativ lange Lagerstabiliät aufzuweisen.

Es ist erfindungsgemäß besonders vorteilhaft, wenn die Emulsion Ethanol enthält.
Dabei ist ein Ethanolgehalt von 1 bis 5 Gew.-%, bezogen auf das Gesamtgewicht der Emulsion erfindungsgemäß bevorzugt.

In diesem Zusammenhang war es besonders überraschend, dass der Schaum trotz Ethanolgehalt in der Emulsion stabil bleibt, da es zum allgemeinen Fachwissen gehört, dass Ethanol Schäume normalerweise destabilisiert. Darüber hinaus weist diese Kombination aus Schaum und Ethanol den überraschenden Vorteil auf, dass der Schaum trotz Gas und Ethanolgehalt nicht entflammbar ist, so dass die üblichen Sicherheitsvorkehrungen für solche Produkte bei der Lagerung, dem Transport und der Anwendung entbehrlich sind.

Es ist erfindungsgemäß vorteilhaft, wenn die Emulsion frei ist von Parabenen, Isothiazolinonen und 3-Iodpropargyl-*N*-butylcarbamat (IPBC) sowie halogenhaltigen Verbindungen.

Hingegen sind erfindungsgemäß vorteilhafte Ausführungsformen dadurch gekennzeichnet, dass die Emulsion Pirocton-Olamin (1-Hydroxy-4-methyl-6-(2,4,4-trimethylpentyl)-2(1*H*)-pyridon monoethanolaminsalz) und/oder Phenoxyethanol enthält.

Enthält die Emulsion Pirocton-Olamin so ist es erfindungsgemäß von Vorteil, wenn der Gehalt an Pirocton-Olamin von 0,01 bis 1 Gew.-%, bezogen auf das Gesamtgewicht der Emulsion beträgt.

Enthält die Emulsion Phenoxyethanol, so ist es erfindungsgemäß von Vorteil, wenn der Gehalt an Phenoxyethanol von 0,1 bis 1 Gew.-%, bezogen auf das Gesamtgewicht der Emulsion beträgt.

Erfindungsgemäß vorteilhafte Ausführungsformen der vorliegenden Erfindung werden auch dadurch erhalten, dass die Emulsion EDTA und/oder Betain enthält.

Enthält die Emulsion EDTA so ist es erfindungsgemäß von Vorteil, wenn der Gehalt an EDTA von 0,01 bis 2 Gew.-%, bezogen auf das Gesamtgewicht der Emulsion beträgt.

Enthält die Emulsion Betain, so ist es erfindungsgemäß von Vorteil, wenn der Gehalt an Betain von 0,001 bis 6 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Lipidphase der Emulsion Fettalkohole (insbesondere Behenylalkohol), Esterwachse, Mandelöl, Kakaobutter und/oder Sheabutter enthält. Dabei ist es insbesondere überraschend gewesen, dass die Emulsionen, wenn sie Behenylalkohol anstatt z.B.Cetearylalkohol, Cetylakohol und/oder Stearylakohol enthält, eine bessere Mischbarkeit mit den Gas(en) Propan, Butan, Isobutan aufweist als Zubereitungen mit diesen Fettalkoholen.

Es ist erfindungsgemäß bevorzugt, wenn die Lipidphase der Emulsion Behenylalkohol, Mandelöl, Kakaobutter und Sheabutter enthält.

Die erfindungsgemäß vorteilhafte Einsatzkonzentration für Behenylalkohol beträgt von 0,01 bis 4 Gew.-%, bezogen auf das Gesamtgewicht der Emulsion.

Die erfindungsgemäß vorteilhafte Einsatzkonzentration für Mandelöl beträgt von 0,01 bis 5 bis Gew.-%, bezogen auf das Gesamtgewicht der Emulsion.

Die erfindungsgemäß vorteilhafte Einsatzkonzentration für Kakaobutter beträgt von 0,01 bis 5 Gew.-%, bezogen auf das Gesamtgewicht der Emulsion.

Die erfindungsgemäß vorteilhafte Einsatzkonzentration für Sheabutter beträgt von 0,01 bis 5 Gew.-%, bezogen auf das Gesamtgewicht der Emulsion.

Erfindungsgemäß vorteilhafte Ausführungsformen der vorliegenden Erfindung sind dadurch gekennzeichnet, dass der Gewichtsanteil der Lipidphase an der Emulsion 7 bis 25 Gew.-%, bezogen auf das Gesamtgewicht der Emulsion beträgt.

Es ist erfindungsgemäß von Vorteil, wenn die Emulsion weder Mineralöl noch Silikonöl enthält.

Silikonöle können jedoch auch in Mengen von kleiner 1 Gew.-%, bezogen auf das Gesamtgewicht der Emulsion enthalten sein.

Die Wasserphase der erfindungsgemäßen Emulsion kann vorteilhaft übliche kosmetische Hilfsstoffe enthalten, wie beispielsweise Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß bevorzugt ist es dabei, wenn der kosmetische Schaum dadurch gekennzeichnet ist, dass die Emulsion, bezogen auf das Gesamtgewicht der Emulsion 5 bis 15 Gew.-% Glycerin enthält.

Darüber hinaus kann die erfindungsgemäße Emulsion vorteilhaft Salze enthalten, insbesondere Meersalz.

Es ist erfindungsgemäß vorteilhaft, wenn die erfindungsgemäße Emulsion einen oder mehrere Wirkstoffe gewählt aus der Gruppe der Verbindungen Glycyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, β-Alanin und/oder Licochalcon A, Panthenol, Tocopherol, Tocopherolacetat, Vitamin C, Vitamin C Derivate, Glycyrrhiza Inflata Root Extract, Magnolienextrakt enthält.

Außerdem sind erfindungsgemäß vorteilhafte Ausführungsformen der vorliegenden Erfindung dadurch gekennzeichnet, dass die Zubereitung Propylenglycol, Butylenglycol, 2-Methylpropan-1,3-diol, Ethylhexylglycerin, 1,2-Pentandiol, 1,2-Hexandiol, 1,2-Octandiol und/oder 1,2-Decandiol enthält. Dabei ist der Gehalt an 2-Methylpropan-1,3-diol, 1,2-Pentandiol und/oder 1,2-Hexandiol erfindungsgemäß bevorzugt.

Erfindungsgemäß vorteilhafte Ausführungsformen der vorliegenden Erfindung sind dadurch gekennzeichnet, dass die Emulsion einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Titandioxid; Zinkoxid.

Es ist erfindungsgemäß besonders bevorzugt, wenn die Emulsion frei ist von Propyl- und Butylparaben, 3-Iod-2-propinylbutylcarbamat, 3-(4-Methylbenzyliden)-campher und 2-Hydroxy-4-methoxybenzophenon (Oxybenzon) und halogenhaltigen Verbindungen.

Die erfindungsgemäßen kosmetischen Emulsionen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben und/oder Aerosile® (CAS-Nr. 7631-86-9) und /oder Talkum, Polymethylsilsesquioxane, Nylon, Silica Dimethyl Silyate

Erfindungsgemäß bevorzugt ist es, wenn der kosmetische Schaum dadurch gekennzeichnet ist, dass die Emulsion mit Polymethylsilsequioxan modifizierte Tapiokastärke (INCI Tapioca starch + Polymethylsilsesquioxane) enthält. Diese wird erfindungsgemäß vorteilhaft in einer Konzentration von 0,1 bis 3 Gew.-%, bezogen auf das Gesamtgewicht der Emulsion eingesetzt.

Der erfindungsgemäße Schaum bzw. die erfindungsgemäße Emulsion haben erfindungsgemäß vorteilhafte einen pH-Wert von 5 bis 7,5.

Erfindungsgemäß vorteilhaft wird der erfindungsgemäße Schaum in einer Aerosoldose mit Entnahmeventil aufbewahrt und aus dieser heraus angewendet. Zur Anwendung wird die erfindungsgemäße Mischung aus Emulsion und Gas(en) durch Schütteln in der Aerosoldose zuerst durchmischt und anschließend über das Entnahmeventil entnommen und auf die Haut appliziert. Dabei zeigt sich ein weiterer erfindungsgemäßer Vorteil, der darin liegt, dass der erfindungsgemäße Schaum sich im Vergleich zu herkömmlichen Schäumen vollständiger aus der Aerosoldose entnehmen lässt, d.h. dass die so genannte "Resteentleerung" überraschend höher ist als bei vergleichbaren, herkömmlichen Schäumen. Darüber hinaus zeigt der erfindungsgemäße Schaum auch bei niedrigen pH-Werten eine überraschend gute Packmittel-Kompatibilität, d.h. dass beispielsweise Korrosionseffekte deutlich seltener und schwächer ausfallen als bei vergleichbaren, herkömmlichen Systemen.

Als Aerosoldosen mit Entnahmeventil können die üblichen für kosmetische Zwecke bekannten Aerosoldosen-Systeme eingesetzt werden.

Erfindungsgemäß ist daher auch eine Aerosoldose mit Entnahmeventil enthaltend den erfindungsgemäßen Schaum, sowie ein Verfahren zur Anwendung kosmetischer Schäume auf der Haut, welches dadurch gekennzeichnet ist, dass die erfindungsgemäße Mischung aus Emulsion und Gas(en) durch Schütteln in der Aerosoldose zuerst durchmischt und anschließend über das Entnahmeventil entnommen und auf die Haut appliziert wird.

Nicht zuletzt ist es erfindungsgemäß bevorzugt, wenn der erfindungsgemäße Schaum dadurch gekennzeichnet ist, dass er in einer Aerosoldose mit Entnahmeventil enthalten ist.

**Vergleichsversuch**

| | **A** | **B** |
|---|---|---|
| **Zusammensetzung in %** | **m [%]** | **m [%]** |
| Glycerylstearatcitrat (Imwitor 372 P) | | 1,75 |
| PEG-40 Stearat | 1,75 | |
| Pirocton-Olamin | 0,06 | 0,06 |
| C15-19 Alkan | 3,00 | 3,00 |
| Isopropyl Palmitat | 3,00 | 3,00 |
| Mineralöl | 3,00 | 3,00 |
| Dimethicon | 0,90 | 0,90 |
| Shea Butter | 1,00 | 1,00 |
| C15-19 Alkan | 3,00 | 3,00 |
| mit Polymethylsilsequioxan modifizierteTapiokastärke | 1,00 | 1,00 |
| Parfüm | 0,35 | 0,35 |
| Glycerin | 9,30 | 9,30 |
| Natronlauge zur pH Wert Einstellung (quantum satis) | | |
| Phenoxyethanol | 0,90 | 0,90 |
| Behenyl Alkohol | 2,00 | 2,00 |
| Ethanol | | |
| Natrium EDTA | 0,18 | 0,18 |
| Wasser | ad 100 | ad 100 |
| | | |
| Formelstabilität nach 1 monatiger Lagerung bei 40°C | Wasserabscheidung (5 mm) | stabil |
| Formelstabilität nach 2 monatiger Lagerung bei 40°C | Deutliche Wasserabscheidung (10 mm) | stabil |

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **C** | **D** | **E** | **F** |
|---|---|---|---|---|
| **Zusammensetzung in %** | **m [%]** | **m [%]** | **m [%]** | **m [%]** |
| Glycerylstearatcitrat (Imwitor 372 P) | 2 | 2 | 2 | 2 |
| Isopropyl Stearat | 1 | | | 1 |
| Caprylic/Capric Triglycerid | 3 | 6 | 3 | 3 |
| Octyldodecanol | 4 | | 3 | 4 |
| Isopropyl Palmitat | | 3 | 3 | |
| Mandelöl | 0,5 | | | |
| Dimethicon | | 1 | | |
| Shea Butter | 1 | 1 | 0,5 | 1 |
| Kakaobutter | | | 0,5 | |
| Tocopherolacetat | | | 0,2 | |
| Meersalz | | 0,1 | | |
| mit Polymethylsilsequioxan modifizierteTapiokastärke | 1 | | 1 | |
| Parfüm | 0,3 | 0,35 | 0,3 | 0,35 |
| Glycerin | 5 | 7 | 9 | 10 |
| Carbomer | 0,1 | 0,2 | 0,05 | 0,1 |
| Phenoxyethanol | 0,8 | 0,6 | 1,0 | 0,75 |
| Pirocton-Olamin | | 0,05 | | |
| Behenyl Alkohol | 2 | 2 | 2 | 2 |
| Ethanol | 1 | 2 | 3 | 1 |
| Natrium EDTA | 0,18 | 0,18 | 0,18 | 0,18 |
| NaOH q.s. auf pH Wert | pH 5,5 | pH 7,0 | pH 7,0 | pH 6,5 |
| Wasser | ad 100 | ad 100 | ad 100 | ad 100 |
| Befüllung in 200 ml Dose | 94% Emulsion | 94% Emulsion | 94% Emulsion | 94% Emulsion |
| Treibmittel | 6% Propan/Butan/I sobutan | 6% Propan/Butan/I sobutan | 6% Propan/Butan/I sobutan | 6% Propan/Butan/I sobutan |
| Druckstufe Gasgemisch | 2.7 | 2.7 | 2.7 | 2.7 |

| | **G** | **H** | **I** | **K** |
|---|---|---|---|---|
| **Zusammensetzung in %** | **m [%]** | **m [%]** | **m [%]** | **m [%]** |
| Glycerylstearatcitrat (Imwitor 372 P) | 1 | 1,5 | 1,75 | 2,5 |
| Sonnenblumenöl | 3 | | | |
| Caprylic/Capric Triglycerid | 4 | 8 | | |
| Octyldodecanol | | | 5 | |
| Isopropyl Palmitat | | | 4 | |
| Mineralöl | | 3 | | |
| Triisostearin | | | | 4 |
| Shea Butter | | | | 2 |
| Stearylakohol | 1 | 0,5 | | |
| Cetylalkohol | 1 | | 2 | 1 |
| Parfüm | | | | |
| Glycerin | 7 | 8 | 5 | 12 |
| Carbomer | 0,1 | | 0,1 | 0,15 |
| Acrylates/C10-30 Alkyl Acrylate Crosspolymer | | 0,15 | 0,1 | |
| Phenoxyethanol | 0,75 | 0,5 | 0,7 | 0,5 |
| 2-Methylpropan-1,3-diol | 2 | 4 | | 2 |
| Pirocton-Olamin | 0,1 | | 0,1 | |
| Ethanol | | | 3 | 2 |
| Natrium EDTA | 0,18 | 0,18 | 0,18 | 0,18 |
| Natronlauge zur pH Wert Einstellung (quantum satis) | pH 5,5 | pH 7,0 | pH 7,0 | pH 6,5 |
| Wasser | ad 100 | ad 100 | ad 100 | ad 100 |
| Befüllung in 200 ml Dose | 96 | 94 | 92 | 90 |
| Treibmittel | 4 Propan/Butan/I sobutan | 6 Propan/Butan/I sobutan | 8 Propan/Butan/I sobutan | 10 Propan/Butan/I sobutan |
| Druckstufe Gasgemisch | 2,7 | 3,0 | 3,2 | 2,7 |

| | **L** | **M** | **N** | **O** |
|---|---|---|---|---|
| **Zusammensetzung in %** | **m [%]** | **m [%]** | **m [%]** | **m [%]** |
| Glycerylstearatcitrat | 2 | 2 | 2 | 2 |
| C12-15 Alkylbenzoat | 3 | 5 | | |
| Dibutyladipate | 2 | | 4 | |
| Diethylamino Hydroxybenzoyl Hexyl Benzoate | | | | 3 |
| Butyl Methoxydibenzoylmethan | 4 | 3 | 4 | |
| Phenylbenzimidazol Sulfonsäure | 2 | 1 | | 2 |
| Ethylhexyl Salicylat | | 5 | 5 | 5 |
| Octocrylen | 5 | | 8 | |
| Parfüm | 0,3 | 0,4 | 0,25 | 0,5 |
| Glycerin | 7 | 8 | 10 | 5 |
| Acrylates/C10-30 Alkyl Acrylate Crosspolymer | 0,2 | 0,1 | 0,2 | 0,15 |
| Phenoxyethanol | 0,7 | 0,4 | 0,8 | 0,9 |
| Ethylhexylglycerin | 0,2 | 0,4 | 0,5 | 0,3 |
| Ethanol | 1 | 2 | 3 | 2 |
| Natrium EDTA | 0,2 | 0,2 | 0,2 | 0,2 |
| Natronlauge zur pH Wert Einstellung (quantum satis) | pH 7,3 | pH 7,5 | pH 7,4 | pH 7,5 |
| Wasser | ad 100 | ad 100 | ad 100 | ad 100 |
| Befüllung in 200 ml Dose | 94 | 94 | 94 | 94 |
| Treibmittel | 6 Propan/Butan/I sobutan | 6 Propan/Butan/I sobutan | 6 Propan/Butan/I sobutan | 6 Propan/Butan/I sobtan |
| Druckstufe Gasgemisch | 3,0 | 3,0 | 3,0 | 3,0 |

## Patentansprüche

1. Kosmetischer Schaum aus
a) einer Emulsion enthaltend Glycerylstearatcitrat und
b) einem die Emulsion aufschäumenden Gas oder Gasgemisch aus Propan, n-Butan und/oder Isobutan,
wobei die Emulsion frei ist von Polyethylenglycol-Derivaten (PEG-Derivaten), d.h. Verbindungen mit Alkohol- oder Säurofunktion, die mit Polyethylenglycolen ganz oder teilweise verethert oder verestert sind, **dadurch gekennzeichnet, dass** die Emulsion frei ist von Seifen und Tensiden mit einem HLB-Wert von größer 12.

2. Kosmetischer Schaum nach Anspruch 1, **dadurch gekennzeichnet, dass** der Schaum gebildet wird aus 90∼96 Gew.-% Emulsion und 4 bis 10 Gew.-% Gas bzw. Gasgemisch.

3. Kosmetischer Schaum nach einem der vorhergehenden Ansprüche, **dadurch gekennzeichnet, dass** die Emulsion frei ist von Parabenen, Isothiazolinonen und 3-Iodpropargyl-*N*-butylcarbamat (IPBC) sowie halogenhaltigen Verbindungen.

4. Kosmetischer Schaum nach einem der vorhergehenden Ansprüche, **dadurch gekennzeichnet, dass** die Emulsion Ethanol enthält.

5. Kosmetischer Schaum nach einem der vorhergehenden Ansprüche, **dadurch gekennzeichnet, dass** die Emulsion Glycerylstearatcitrat in einer Menge von 0,1 bis 5 Gew.-%, bezogen auf das Gesamtgewicht der Emulsion, enthält.

6. Kosmetischer Schaum nach einem der vorhergehenden Ansprüche, **dadurch gekennzeichnet, dass** dfe Emulsion Pirocton-Olamin (1-Hydroxy-4-methyl-6-(2,4,4-trimethylpentyl)-2(1*H*-pyridon monoethanolaminsalz) und/oder Phenoxyethanol enthärt.

7. Kosmetischer Schaum nach einem der vorhergehenden Ansprüche, **dadurch gekennzeichnet, dass** die Emulsion EDTA und/oder Betain enthält.

8. Kosmetischer Schaum nach einem der vorhergehenden Ansprüche, **dadurch gekennzeichnet, dass** die Lipidphase der Emulsion Behenylalkohol, Mandelöl, Kakaobutter und/oder Sheabutter enthält.

9. Kosmetischer Schaum nach einem der vorhergehenden Ansprüche, **dadurch gekennzeichnet, dass** der Gewichtsanteil der Lipidphase an der Emulsion 7 bis 25 Gew.-%, bezogen auf das Gesamtgewicht der Emulsion beträgt.

10. Kosmetischer Schaum nach einem der vorhergehenden Ansprüche, **dadurch gekennzeichnet, dass** die Emulsion weder Mineralöl noch Silikonöl enthält.

11. Kosmetischer Schaum nach einem der vorhergehenden Ansprüche, **dadurch gekennzeichnet, dass** die Emulsion mit Polymethylsilsequioxan modifizierteTapiokastärke (INCI Tapioca starch + Polymethylsilsesquioxane) enthält.

12. Kosmetischer Schaum nach einem der vorhergehenden Ansprüche, **dadurch gekennzeichnet, dass** die Emulsion, bezogen auf das Gesamtgewicht der Emulsion 5 bis 15 Gew.-% Glycerin enthält.

13. Aerosoldose mit Entnahmeventil enthaltend einen kosmetischen Schaum nach einem der Ansprüche 1 bis 12.

## Claims

1. Cosmetic foam composed of
a) an emulsion comprising glyceryl stearate citrate and
b) a gas or gas mixture composed of propane, n-butane and/or isobutane that foams up the emulsion,
wherein the emulsion is free of polyethylene glycol derivatives (PEG derivatives), i.e. compounds having alcohol or acid function that have been wholly or partly etherified or esterified with polyethylene glycols, **characterized in that** the emulsion is free of soaps and surfactants having an HLB value of greater than 12.

2. Cosmetic foam according to Claim 1, **characterized in that** the foam is formed from 90-96% by weight of emulsion and 4-10% by weight of gas or gas mixture.

3. Cosmetic foam according to either of the preceding claims, **characterized in that** the emulsion is free of parabens, isothiazolinones and 3-iodopropargyl *N*-butylcarbamate (IPBC) and halogenated compounds.

4. Cosmetic foam according to any of the preceding claims, **characterized in that** the emulsion contains ethanol.

5. Cosmetic foam according to any of the preceding claims, **characterized in that** the emulsion contains glyceryl stearate citrate in an amount of 0.1% to 5% by weight, based on the total weight of the emulsion.

6. Cosmetic foam according to any of the preceding claims, **characterized in that** the emulsion contains piroctone olamine (1-hydroxy-4-methyl-6-(2,4,4-trimethylpentyl)-2(1*H*)-pyridone monoethanolamine salt) and/or phenoxyethanol.

7. Cosmetic foam according to any of the preceding claims, **characterized in that** the emulsion contains EDTA and/or betaine.

8. Cosmetic foam according to any of the preceding claims, **characterized in that** the lipid phase of the emulsion contains behenyl alcohol, almond oil, cocoa butter and/or shea butter.

9. Cosmetic foam according to any of the preceding claims, **characterized in that** the proportion by weight of the lipid phase in the emulsion is 7% to 25% by weight, based on the total weight of the emulsion.

10. Cosmetic foam according to any of the preceding claims, **characterized in that** the emulsion contains neither mineral oil nor silicone oil.

11. Cosmetic foam according to any of the preceding claims, **characterized in that** the emulsion contains polymethylsilsesquioxane-modified tapioca starch (INCI Tapioca starch + polymethylsilsesquioxane).

12. Cosmetic foam according to any of the preceding claims, **characterized in that** the emulsion, based on the total weight of the emulsion, contains 5% to 15% by weight of glycerol.

13. Aerosol can with an outlet valve, containing a cosmetic foam according to any of Claims 1 to 12.

## Revendications

1. Mousse cosmétique constituée par
a) une émulsion contenant du citrate de stéarate de glycéryle et
b) un gaz ou un mélange gazeux faisant mousser l'émulsion, constitué par le propane, le n-butane et/ou l'isobutane
l'émulsion étant exempte de dérivés de polyéthylèneglycol (dérivés de PEG) c'est-à-dire de composés présentant une fonction alcool ou acide, totalement ou partiellement éthérifiés ou estérifiés avec des polyéthylèneglycols, **caractérisée en ce que** l'émulsion est exempte de savons et de tensioactifs présentant une valeur BHL supérieure à 12.

2. Mousse cosmétique selon la revendication 1, **caractérisée en ce que** la mousse est formée à partir de 90-96 % en poids d'émulsion et de 4 à 10 % en poids de gaz ou de mélange gazeux.

3. Mousse cosmétique selon l'une quelconque des revendications précédentes, **caractérisée en ce que** l'émulsion est exempte de parabènes, d'isothiazolinones et de N-butylcarbamate de 3-iodopropargyle (IPBC) ainsi que de composés halogénés.

4. Mousse cosmétique selon l'une quelconque des revendications précédentes, **caractérisée en ce que** l'émulsion contient de l'éthanol.

5. Mousse cosmétique selon l'une quelconque des revendications précédentes, **caractérisée en ce que** l'émulsion contient du citrate de stéarate de glycéryle en une quantité de 0,1 à 5 % en poids, par rapport au poids total de l'émulsion.

6. Mousse cosmétique selon l'une quelconque des revendications précédentes, **caractérisée en ce que** l'émulsion contient de l'olamine de piroctone (sel de monoéthanolamine de 1-hydroxy-4-méthyl-6-(2,4,4-triméthylpentyl)-2(1H)-pyridone, et/ou du phénoxyéthanol.

7. Mousse cosmétique selon l'une quelconque des revendications précédentes, **caractérisée en ce que** l'émulsion contient de l'EDTA et/ou de la bétaïne.

8. Mousse cosmétique selon l'une quelconque des revendications précédentes, **caractérisée en ce que** la phase lipidique de l'émulsion contient de l'alcool béhénylique, de l'huile d'amande, du beurre de cacao et/ou du beurre de karité.

9. Mousse cosmétique selon l'une quelconque des revendications précédentes, **caractérisée en ce que** la proportion pondérale de la phase lipidique par rapport à l'émulsion est de 7 à 25 % en poids, par rapport au poids total de l'émulsion.

10. Mousse cosmétique selon l'une quelconque des revendications précédentes, **caractérisée en ce que** l'émulsion ne contient ni huile minérale, ni huile de silicone.

11. Mousse cosmétique selon l'une quelconque des revendications précédentes, **caractérisée en ce que** l'émulsion contient de l'amidon de tapioca modifié par du polyméthylsilsesquioxane (INCI Tapioca starch + Polymethylsilsesquioxane).

12. Mousse cosmétique selon l'une quelconque des revendications précédentes, **caractérisée en ce que** l'émulsion contient, par rapport au poids total de l'émulsion, 5 à 15 % en poids de glycérol.

13. Bombe à aérosol présentant une soupape de prélèvement contenant une mousse cosmétique selon l'une quelconque des revendications 1 à 12.
